# EUROPEAN PATENT SPECIFICATION

(11) **EP 0 357 787 B1**
(45) Date of publication and mention of the grant of the patent: **08.05.1996**
(21) Application number: 89902081.2
(22) Date of filing: 07.02.1989
(51) Int. Cl.: C12P 7/62

(54) **PROCESS FOR PREPARING OPTICALLY ACTIVE 2-HYDROXY ACID DERIVATIVES**
VERFAHREN ZUR HERSTELLUNG VON OPTISCH AKTIVEN 2-HYDROXYSÄUREABKÖMMLINGEN
PROCEDE DE PREPARATION DE DERIVES DE L'ACIDE 2-HYDROXY OPTIQUEMENT ACTIFS

(30) Priority: 12.02.1988 JP 30476/88; 12.02.1988 JP 30477/88; 28.04.1988 JP 105892/88; 30.04.1988 JP 109938/88
(43) Date of publication of application: 14.03.1990
(73) Proprietor: DAICEL CHEMICAL INDUSTRIES, LTD., Sakai-shi Osaka-fu 590 (JP)
(72) Inventor: MATSUYAMA, Akinobu, Niigata 944 (JP); NIKAIDO, Teruyuki, Niigata 944 (JP); KOBAYASHI, Yoshinori, Niigata 942 (JP)
(74) Representative: Grünecker, Kinkeldey, Stockmair & Schwanhäusser Anwaltssozietät
(86) International application number: JP8900121
(87) International publication number: WO8907648

(56) References cited:
- EP-A- 0 024 547
- EP-A- 0 130 288
- EP-A- 0 137 301
- EP-A- 0 218 863
- JP-A- 6 261 587
- JP-A-59 162 894

## Description

### Technical Field

This invention relates to a process for the production of an optically active 2-hydroxy acid derivative, for example, an ester. More particularly, it relates to a process for the production of an optically active 2-hydroxy acid derivative which comprises treating a 2-oxo acid derivative represented by the formula (I) with a microorganism as mentioned in claim 1, which may be optionally ground, treated with aceton, lyophilized or immobilized, capable of asymmetrically reducing said 2-oxo acid derivative of the formula (I) into an optically active (R)- or (S)-2-hydroxy acid derivative represented by the formula (II) and recovering the optically active (R)- or (S)-2-hydroxy acid derivative of the formula (II) thus formed.

Optically active 2-hydroxy acid derivatives represented by the formula (II) are important intermediates in the synthesis of various drugs, for example, a remedy for hypertension.

### Background Art

Known methods for the production of optically active ethyl 2-hydroxy-4-phenylbutyrate, which is one of 2-hydroxy acid derivatives represented by the formula (II), include chemical and asymmetric reduction of (R)-2-hydroxy-4-phenylbutyric acid (cf. Eur. Pat. EP 206993) and chemical synthesis comprising synthesizing (R)-2-hydroxy-4-phenylbutyric acid from benzylmagnesium chloride and optically active glycidic acid followed by ethyl-esterification (cf. Japanese Patent Laid-Open No. 212329/1987).

However the former method comprising the asymmetric chemical reduction cannot give a satisfactory optical purity of the product, while the latter one comprising the chemical synthesis is disadvantageous in that optically active serine, which is the starting material for the optically active glycidic acid, is expensive for industrial uses.

Furthermore, no process for the production of an optically active 2-hydroxy acid derivative represented by the formula (II) from a 2-oxo acid derivative represented by the formula (I) by taking advantage of the capability of asymmetric reduction of a microorganism has been reported so far. Disclosure of Invention

The present inventors have paid attention to a process for conveniently producing an optically active 2-hydroxy acid derivative represented by the formula (II) having a high optical purity through asymmetric reduction with a microorganism and attempted to find out microorganisms suitable for the above purpose. As a result, they have found that microorganisms belonging to the genera Streptococcus, Guilliermondella, Candida, Saccharomycopsis, Zygosaccharomyces, Sporidiobolus, Rhodosporidium, Saccharomyces,Schizosaccharomyces, Pichia, Issatchinkia, Rhodotorula, Kluyveromyces, Filobasidium, Torulaspora, Sporobolomyces, Hansenula, Lipomyces, Lodderomyces, Pachysolen, Saccharomycodes, Achromobacter, Brevibacterium, Erwinia, Klebsiella, Psudomonas, Bacillus, Xanthomonas, Torulopsis and Chromobacterium could asymmetrically reduce a 2-oxo acid derivative represented by the formula (I) to thereby give an optically active (R)-2-hydroxy acid derivative represented by the formula (II) ; and that microorganisms belonging to the genera Streptococcus, Sporolactobacillus, Ambrosiozima, Botryoascus, Bretanomyces, Clavispora, Candida, Saccharomyces, Zygosaccharomyces, Schizosaccharomyces, Saccharomycopsis, Sporobolomyces, Rhodotorula, Pichia, Hansenula, Syringospora, Stephanoascus, Trigonopsis, Wickerhamiella, Wingea, Schwanniomyces, Geotrichum, Ashybya, Endomyces, Alcaligenes, Escherichia, Serratia, Pseudomonas, Pimelobacter, Bacillus, Brevibacterium, Staphylococcus, Aureobacterium, Flavobacterium, Paracoccus, Citrobacter, Protaminobacter, Rhodococcus, Micrococcus, Agrobacterium, Corynebacterium, Mycobacterium and Arthrobacter could asymmetrically reduce a 2-oxo acid derivative represented by the formula (I) to thereby give an optically active (S)-2-hydroxy acid derivative represented by the formula (II), thus completing the present invention.

The 2-oxo acid derivative to be used as the starting material in the present invention is represented by the formula (I). Examples thereof include methyl, ethyl, propionyl and butyl esters of benzoylformic, phenylpyruvic, 2-oxo-4-phenylbutyric and 2-oxo-5-phenylvaleric acids.

In the present invention, any microorganism belonging to the genus Streptococcus, Guilliermondella, Candida, Saccharomycopsis, Zygosaccharomyces, Sporidiobolus, Rhodosporidium, Saccharomyces, Schizosaccharomyces, Pichia, Issatchinkia, Rhodotorula, Kluyveromyces, Filobasidium, Torulaspora, Sporobolomyces, Hansenula, Lipomyces, Lodderomyces, Pachysolen, Saccharomycodes, Achromobacter, Brevibacterium, Erwinia, Klebsiella. Pseudomonas. Bacillus, Xanthomonas, Torulopsis and Chromobacterium and capable of asymmetrically reducing a 2-oxo acid derivative represented by the formula (I) into an optically active (R)-2-hydroxy acid derivative represented by the formula (II) or one belonging to the genus Streptococcus, Sporolactobacillus, Ambrosiozyma, Botryoascus, Bretanomyces, Clavispora, Candida, Saccharomyces, Zygosaccharomyces, Schizosaccharomyces, Saccharomycopsis, Sporobolomyces, Rhodotorula, Pichia, Hansenula, Syringospora, Stephanoascus, Trigonopsis, Wickerhamiella, Wingea, Schwanniomyces, Geotrichum, Ashybya, Endomyces, Alcaligenes, Escherichia, Serratia, Pseudomonas, Pimelobacter, Bacillus, Brevibacterium, Staphylococcus, Aureobacterium, Flavobacterium, Paracoccus, Citrobacter, Protaminobacter, Rhodococcus, Micrococcus, Agrobacterium, Corynebacterium, Mycobacterium or Arthrobacter capable of asymmetrically reducing a 2-oxo acid derivative represented by the formula (I) into an optically active (S)-2-hydroxy acid derivative represented by the formula (I) may be used.

Particular examples of the microorganism capable of producing an optically active (R)-2-hydroxy acid derivative represented by the formula (II) from a 2-oxo acid derivative represented by the formula (I) include Streptococcus alactosus NRIC1154 and ATCC8058, Streptococcus equinus NRIC1139 and ATCC9812, Streptococcus faecium NRIC1145 and ATCC19434, Streptococcus uberis NRIC1153 and ATCC19436, Guilliermondella selenospora IFO1850, Candida guilliermondii IAM4412, Saccharomycopsis fibuligera IFO0103, Saccharomycopsis capsularis IFO0672, Zygosaccharomyces bailii IFO1047, Sporidiobolus pararoseus AHU3447, Rhodosporidium diobovatum IFO0682, Rhodosporidium toruloides IFO0559, Saccharomyces rouxii IAM4011, Saccharomyces dairensis IFO0285, Torulaspora delbrueckii IFO0955, Schizosaccharomuyces pombe IFO0363, Pichia heedii IFO10019, Pichia membranaefaciens IFO0577, Pichia opuntiae var. thermotolerans IFO10024, Issatchinkia scutulata var scutulata IFO10069, Rhodotorula rubra AHU3243, Rhodotorula glutinis AHU3454, Kluyveromyces lactis IFO1267, Kluyveromyces drosphilarum IFO1012, Filobasidium capsuligenum IFO1185, Torulaspora delbrueckii IFO0381, Sporobolomyces rosenus IFO1037, Hansenula holsttii IFO0980, Hansenula subpelliculosa IFO0808, Sporidiobolus johnsonii IFO6903, Lipomyces starkeyi IFO1289, Lodderomyces elongisporus IFO1676, Pachysolen tannophilus IFO1007, Saccharomycodes ludwigii IFO0798, Achromobacter pestifer ATCC23584, Brevibacterium iodinum IF03558, Erwinia carotovora IFO3880, Klebsiella pneumoniae IAM1063, Pseudomonas dacunhae IFO12048, Bacillus licheniformis IFO12200, Bacillus cereus IFO3001 and Xanthomonas oryzae IAM1657.

Examples of the microorganism capable of producing an optically active (S)-2-hydroxy acid derivative represented by the formula (II) from a 2-oxo acid derivative represented by the formula (I) include Streptococcus agalactiae NRIC1137 and ATCC13813, Streptococcus lactis NRIC1149 and ATCC19435, Streptococcus faecalis IFO12964, Sporolactobacillus inulinus NRIC1133 and ATCC15538, Ambrosiozyma cicatricosa IFO1846, Botryoascus synnaedendrus IFO1604, Bretanomyces bruxellensis IFO0268, Clavispora lusitaniae IFO1019, Candida humicola IFO0760, Candida parasilosis IFO1396, Candida pseudotropicalis IAM4829, Candida utilis IAM4220, Candida rugosa IFO0750, Saccharomyces bayanus IFO0262, Saccharomyces cerevisiae ATCC9080, Saccharomyces kluyveri IFO1893, Saccharomyces uvarum IFO0565, Saccharomyces chevalieri IFO0222, Zygosaccharomyces fermentati IFO0021, Schizosaccharomyces octosporus IFO0353, Saccharomycopsis lipolytica IFO1551, Sporobolomyces salmonicolor AHU3982, Rhodotolura glutinis IF00389, Rhodotorula minuta IF00387, Pichia opuntiae var.thermotolerans IFO10025, Pichia burtonii IFO1986, Pichia farinosa IFO1163, Hansenula fabianii IFO1254, Syringospora albicans IFO1856, Stephanoascus ciferrii IFO1854, Trigonopsis variabilis IFO0755, Wickerhamiella domercqii IFO1857, Wingea robertsii IFO1277, Schwanniomyces occidentalis IFO1841, Geotrichum candidum IFO4601, Ashbya gossypii IFO1355, Endomyces decipiens IFO0102, Alcaligenes faecalis IAM1015, Escherichia coli IFO3544, Serratia marcescens IFO3046, Pseudomonas aureofaciens IFO3522, Pseudomonas fluorescens IFO3925, Pseudomonas riboflavina IFO13584, Pseudomonas chlororaphis IFO3904, Pimelobacter simplex IFO12069, Bacillus subtilis IFO3007, Brevibacterium ammoniagenes IAM1641, Staphylococcus aureus IFO3060, Aureobacterium testaceus IFO12675, Flavobacterium suaveolens IF03752, Paracoccus denitrificans IFO12442, Citrobacter freundii AHU1534, Protaminobacter ruber IAM1081, Rhodococcus equii IFO3730, Micrococcus luteus IFO12992, Agrobacterium radiobacter IFO12664, Corynebacterium glutamicum ATCC13032 and Mycobacterium smegmatis IFO3153.

Each strain may be either a wild type, a variant or a recombinant obtained by genetic engineering such as cell fusion or gene recombination.

Microorganisms having IFO numbers assigned thereto are described in the List of Culture, 8th ed., vol. 1 (1988) published by the Institute for Fermentation, Osaka (IFO) and available therefrom. Those having AHU numbers are described in the Catalogue Culture, 4th ed. (1987) published by Japan Federation of Culture Collection (JFCC) and available from the Faculty of Agriculture, Hokkaido University. Those having ATCC numbers are described in the Catalogue of Bacteria Phages rDNA Vectors, 16th ed. (1985) published by American Type Culture Collection (ATCC) and available therefrom. Those having NRIC numbers are described in the Culture Collection of NODAI No. 1 (1985) published by Tokyo University of Agriculture and available therefrom. Those having IAM numbers are available from the Institute of Applied Microbiology, the University of Tokyo.

The microorganism to be used in the present invention may be cultured in any medium so long as it can grow therein. Any carbon source may be used so long as said microorganism can utilize it. Examples thereof include sugars such as glucose, fructose, sucrose and dextrin, alcohols such as sorbitol, ethanol and glycerol, organic acids such as fumaric, citric, acetic and propionic acids and salts thereof, hydrocarbons such as paraffin and mixtures thereof. Examples of a nitrogen source include ammonium salts of inorganic acids, such as ammonium chloride, ammonium sulfate and ammonium phosphate, ammonium salts of organic acids, such as ammonium fumarate and ammonium citrate, nitrogenous materials such as meat extract, yeast extract, corn steep liquor, casein hydrolysate and urea and mixtures thereof. Furthermore various nutritional sources commonly used in the culture of microorganisms, such as inorganic salts, trace metal salts and vitamins, may be appropriately mixed and used in the present invention. In addition, materials effective in promoting the growth of the microorganism, in elevating the productivity of the target compound or in maintaining the pH value of the medium on the desired level may be added, if required.

The pH value of the medium may be adjusted to 3.0 to 9.5, preferably 5 to 8. The culture may be carried out at a temperature of 20 to 45°C, preferably 25 to 37°C, either aerobically or anaerobically under conditions suitable for the growth of the microorganism for 15 to 120 hours, preferably 12 to 72 hours.

The reduction may be effected by using the culture medium as such. Alternately, the cells may be separated by, for example, centrifugation, optionally washed and resuspended in a buffer solution or water. Then a 2-oxo acid derivative represented by the formula (I) may be added to the suspension thus obtained. In this reaction, it is sometimes preferable to add a carbon source such as glucose or sucrose to the medium to thereby supply energy. The cells may be used as such in the form of viable cells. Alternately, they may be ground, treated with acetone or lyophilized. These cells, which have been optionally treated, may be immobilized prior to the use by a conventional method such as the polyacrylamide gel, carrageenan gel, alginic acid gel or agar gel method. Furthermore, an enzyme obtained from said treated cells by combining known methods may be used in the present invention.

The 2-oxo acid derivative represented by the formula (I) may be added either at once at the initiation of the reaction or by portions either as such, dissolved in water or an inert organic solvent or dispersed in, for example, a surfactant.

The reaction may be conducted at a pH value of 3 to 9, preferably 5 to 8, at 10 to 60°C, preferably 20 to 40°C for 1 to 120 hours with or without stirring. The concentration of the substrate may preferably range from 0.1 to 10%, though it is not restricted thereby.

The optically active (R)- or (S)-2-hydroxy acid derivative represented by the formula (II) thus produced may be readily collected by extracting the reaction mixture, from which the cells may be optionally separated, with an organic solvent and purifying the extract by, for example, column chromatography or recrystallization.

### Best Mode for Carrying Out the Invention

To further illustrate the present invention, and not by way of limitation, the following Examples will be given.

In each Example, the absolute configuration and optical purity were determined by extracting the reaction product with ethyl acetate and subjecting the obtained extract to high performance liquid chromatography by using an optical resolution column [column: Chiral cell OB, mfd. by Daicel Chemical Industries, Ltd., 4.6 mm (i.d.) x 250 mm, solvent: n-hexane : 2-propanpol (19 : 1 v/v), flow rate: 0.5 ml/min, Detection: 254 nm]. The reaction yield was determined by gas chromatography [column: PEG20M, 10%, 2 m, 200°C].

### Example 1

100 ml of a medium comprising 2% of glucose, 0.5% of yeast extract, 0.3% of peptone, 0.3% of meat extract, 0.1% of monopotassium phospate, 0.2% of dipotassium phosphate and 0.5% of calcium carbonate was introduced into a 500-ml Erlenmeyer flask and sterilized. Next, each strain specified in Table 1 was inoculated thereto and cultured therein at 30°C for 30 hours under rotation and shaking.

After the completion of the culture, the cells were separated by centrifugation and washed once with a physiological saline solution to thereby give viable cells.

7.5 ml of distilled water was introduced into a 100-ml Erlenmeyer flask and the above viable cells were suspended therein. 1.2 g of glucose was added to the suspension thus obtained and the resulting mixture was shaken under rotation at 30°C for 10 minutes. Then 0.1 g of ethyl 2-oxo-4-phenylbutyrate and 0.3 g of calcium carbonate were added thereto and the resulting mixture was shaken under rotation at 30°C for 20 hours.

After the completion of the reaction, the optically active ethyl 2-hydroxy-4-phenylbutyrate thus formed was extracted with 20 ml of ethyl acetate

The ethyl acetate phase was analyzed by gas chromatography and the reaction yield was determined. Next, a given amount of the ethyl acetate phase was dehydrated with anhydrous Glauber's salt and the solvent was removed therefrom. The oily product thus obtained was dissolved in ethanol and subjected to high performance liquid chromatography. The absolute configuration and optical purity of the optically active ethyl 2-hydroxy-4-phenylbutyrate thus obtained were determined.

Table 1 shows the results.

### Example 2

100 ml of a YM medium comprising 0.3% of yeast extract, 0.3% of malt extract, 0.5% of peptone and 2% of glucose (pH 6.0) was introduced into a 500-ml Sakaguchi flask and sterilized. Then each strain specified in Table 2 was inoculated thereto and cultured therein at 30°C for 48 hours under shaking.

After the completion of the culture, the cells were separated by centrifugation and washed once with a physiological saline solution to thereby give viable cells.

50 ml of distilled water was introduced into a 500-ml Sakaguchi flask and the abovementioned viable cells were suspended therein. Then 6 g of sucrose was added to the obtained suspension and the mixture was reciprocally shaken at 30°C for 10 minutes. Next, 0.5 g of ethyl 2-oxo-4-phenylbutyrate was added thereto and the resulting mixture was reciprocally shaken at 30°C for 20 hours.

After the completion of the reaction, the reaction yield, absolute configuration and optical purity of the optically active ethyl 2-hydroxy-4-phenylbutyrate thus obtained were determined in the same manner as the one described in Example 1.

Table 2 shows the results.

### Example 3

Each microorganism specified in Table 3 was subjected to the same treatment as that described in Example 1 except that the medium contained no calcium carbonate and had a pH value of 7 and that the culture was reciprocally conducted in a 500-ml Sakaguchi flask.

After the completion of the reaction, the reaction yield, absolute configuration and optical purity of the optically active ethyl 2-hydroxy-4-phenylbutyrate thus obtained were determined in the same manner as the one described in Example 1.

Table 3 shows the results.

### Example 4

Pseudomonas aureofaciens IFO3522 was inoculated into 2-ℓ of the same medium as the one described in Example 1 except containing no calcium carbonate in a 5-ℓ jar fermenter and cultured therein at 30°C under stirring at 400 rpm and aerating at 1 vvm for 30 hours.

After the completion of the culture, the cells were collected by centrifugation and washed with 1-ℓ of water. Then these cells were suspended in 200 ml of water and introduced into a 1-ℓ Erlenmeyer flask. Twenty g of ethyl 2-oxo-4-phenylbutyrate, 20 g of glucose and 2 g of calcium carbonate were added thereto and the obtained mixture was allowed to react at 30°C under stirring for 48 hours.

After the completion of the reaction, the reaction mixture was extracted with 100-ml portions of ethyl acetate twice. The ethyl acetate phase was dehydrated with anhydrous Glauber's salt and the solvent was removed therefrom under reduced pressure. Then it was distilled in a conventional manner under reduced pressure of 0.5 mmHg (b.p.: 115 - 118°C). Thus 0.7 g of the aimed ethyl (S)-2-hydroxy-4-phenylbutyrate was obtained (yield: 35%, optical purity: 96% e.e.).

The process of the present invention for the production of an optically active 2-hydroxy acid derivative through asymmetric reduction by using a microorganism makes it possible to readily produce an optically active 2-hydroxy acid derivative having a high optical purity. Thus it is highly advantageous as an industrial process.

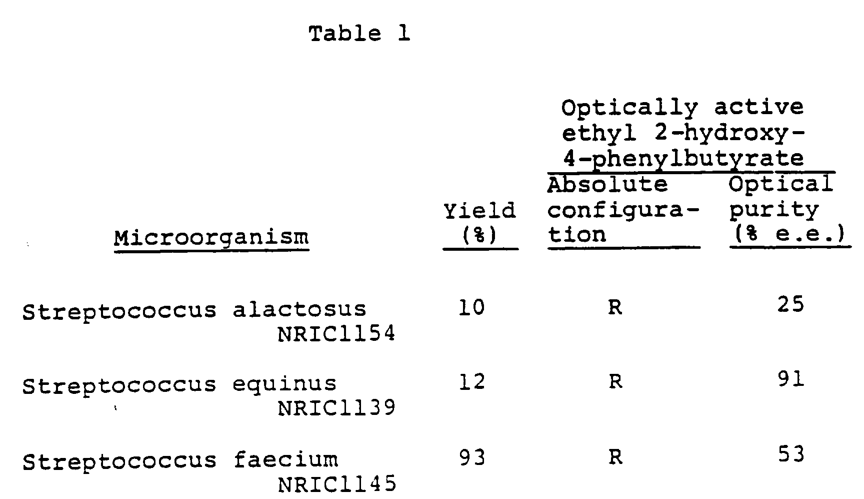

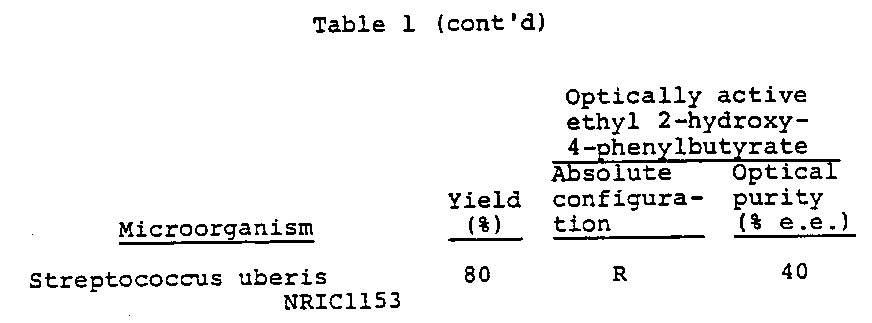

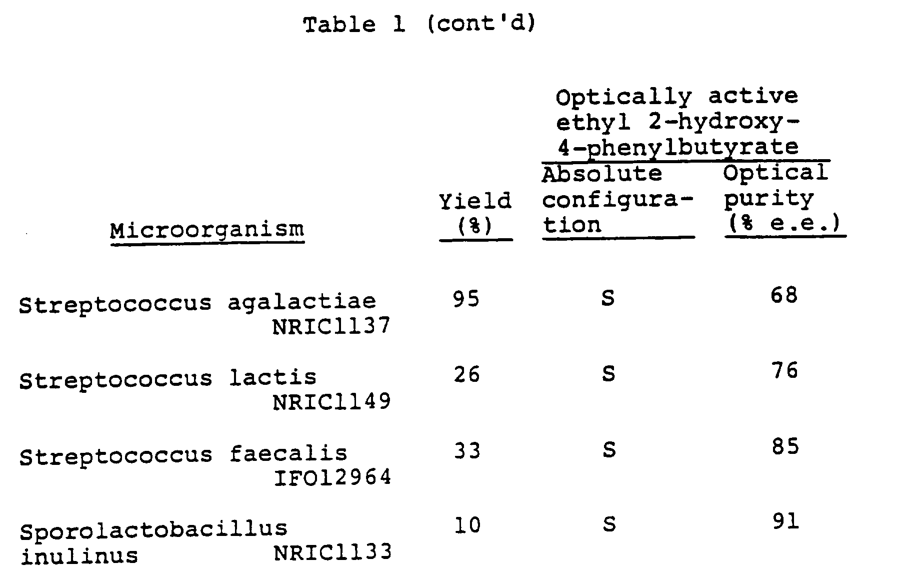

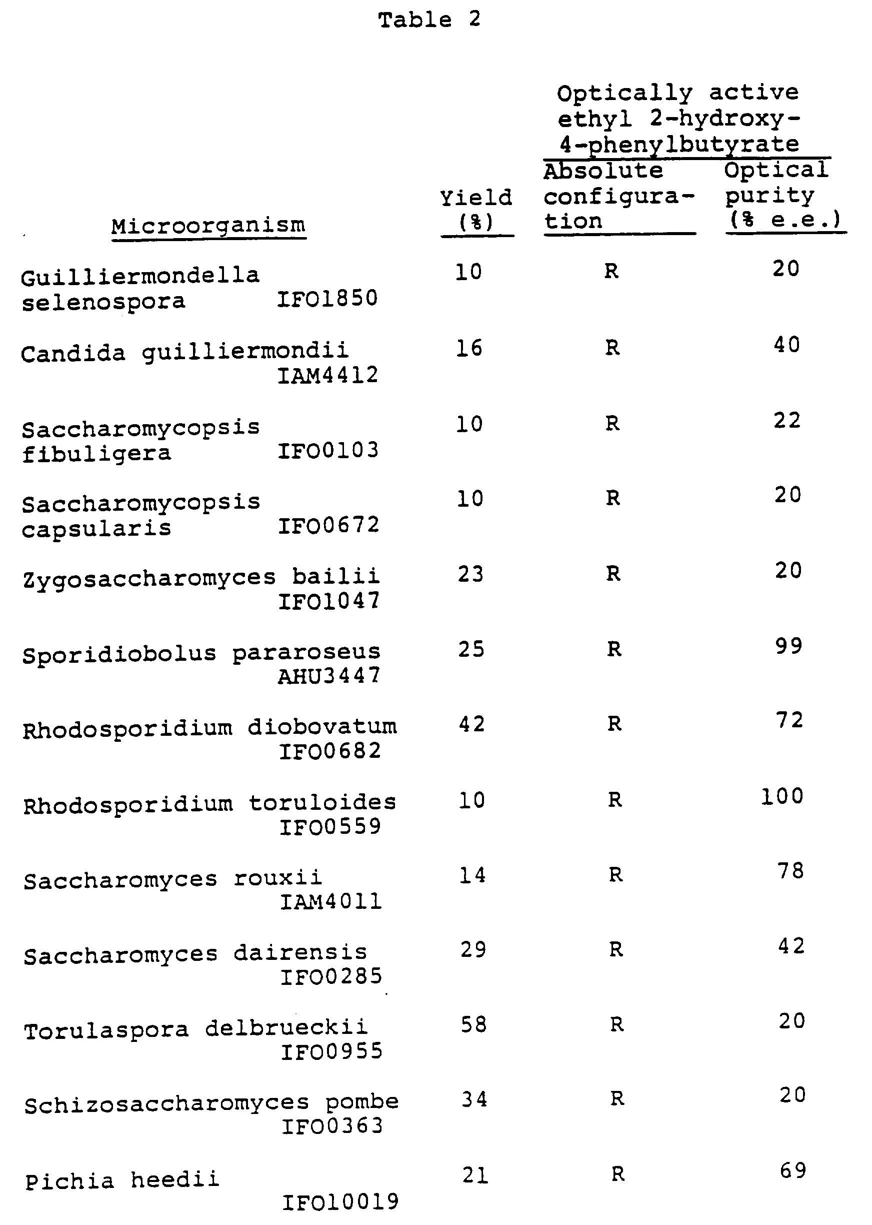

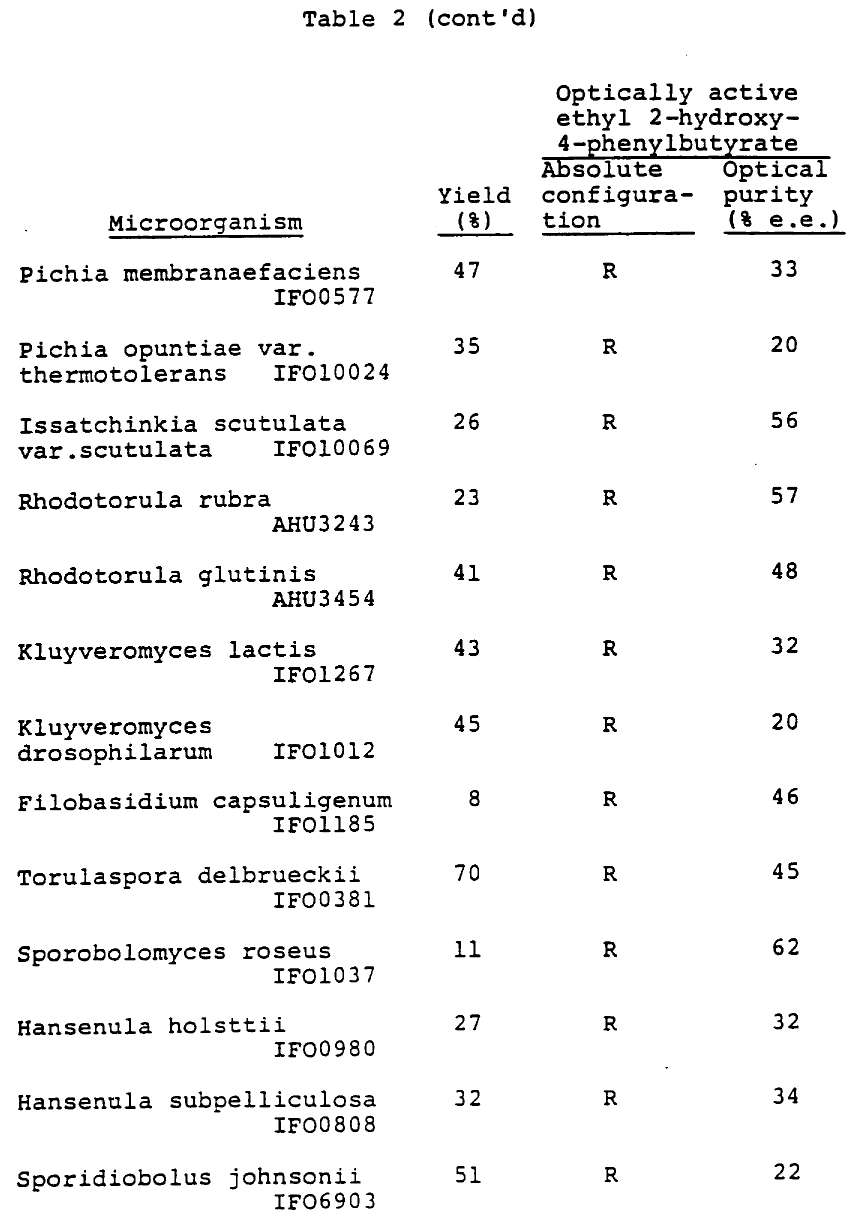

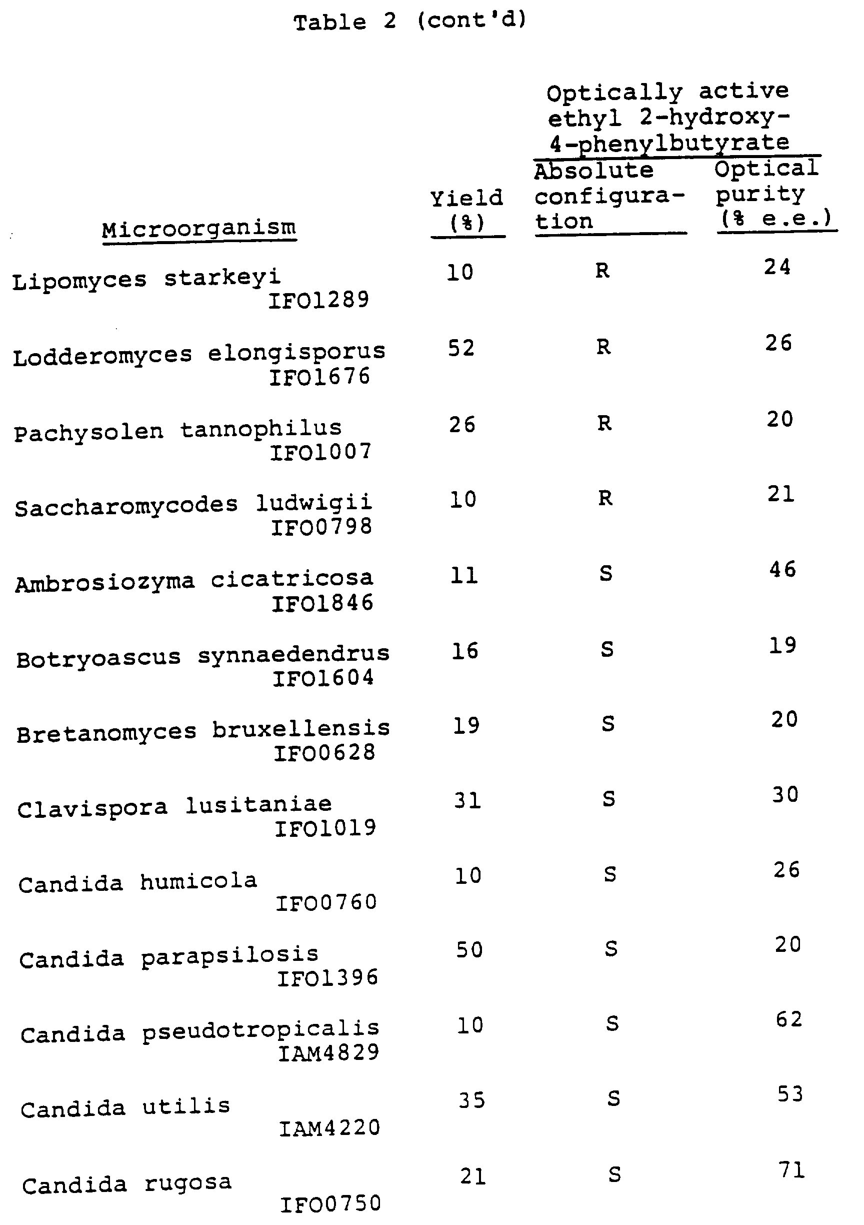

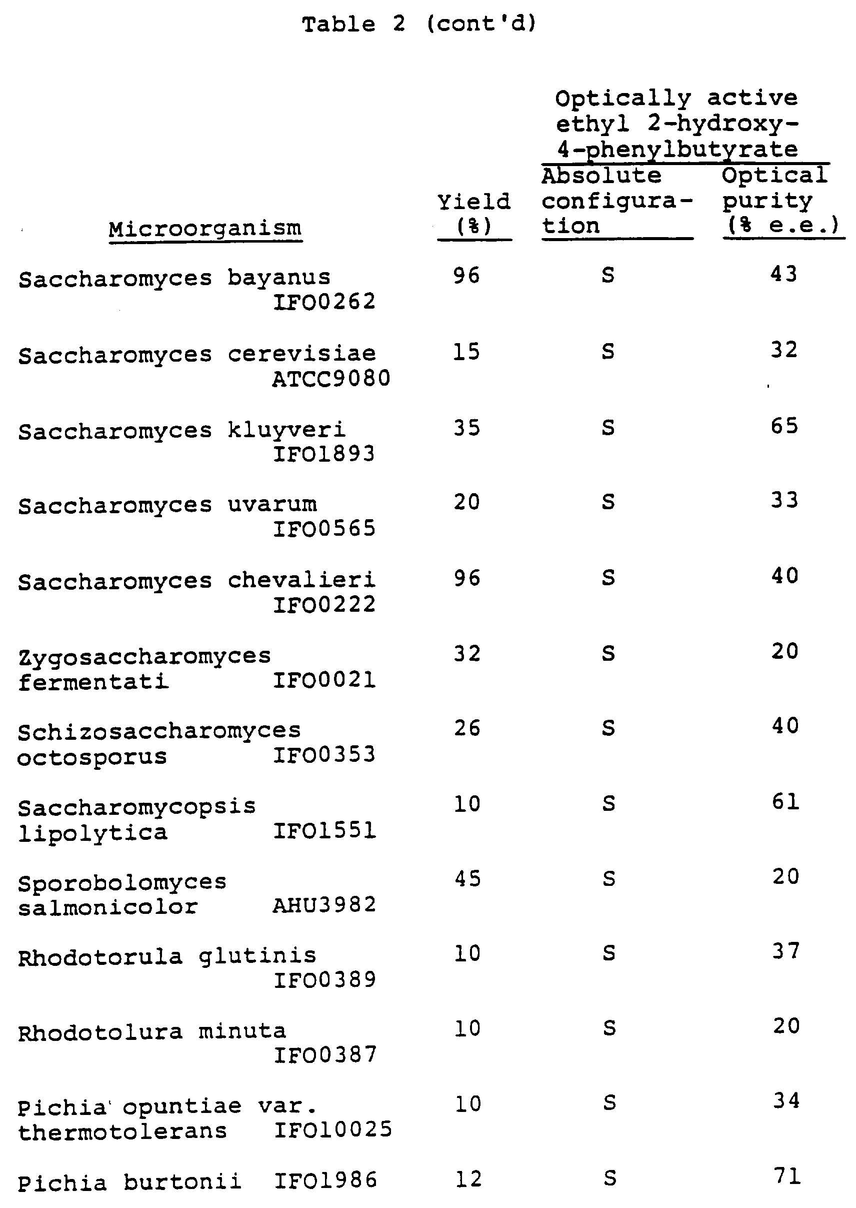

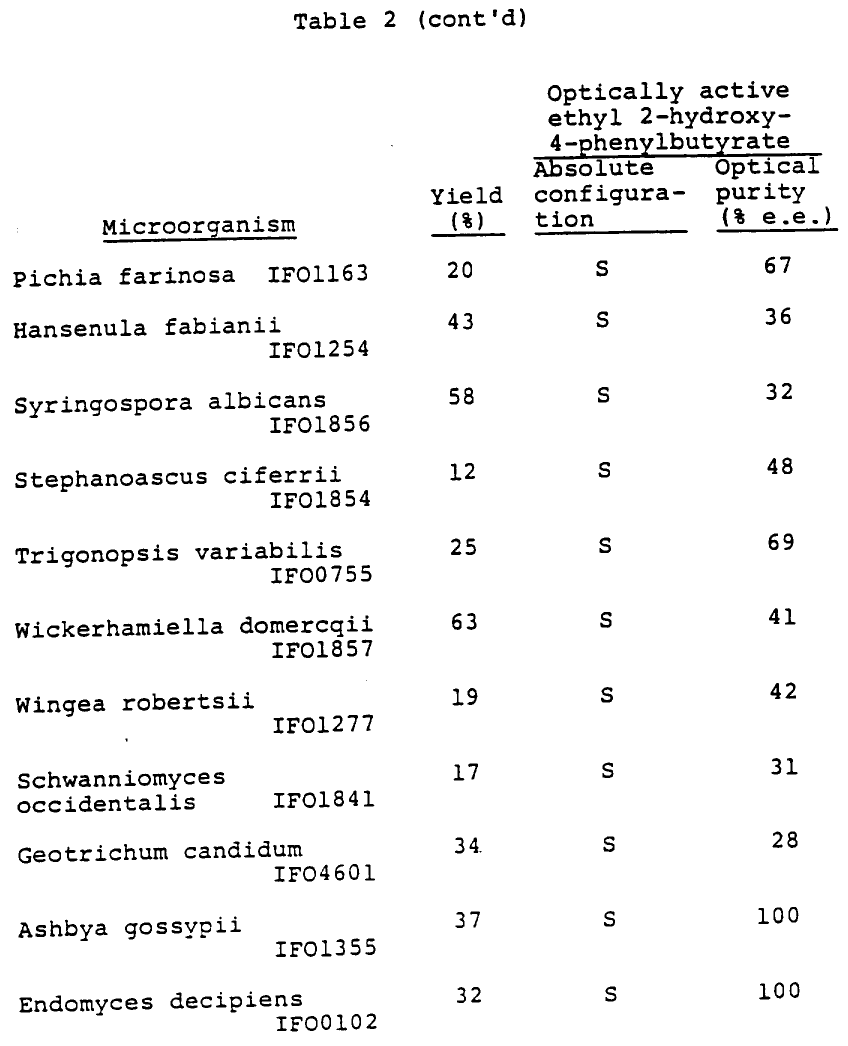

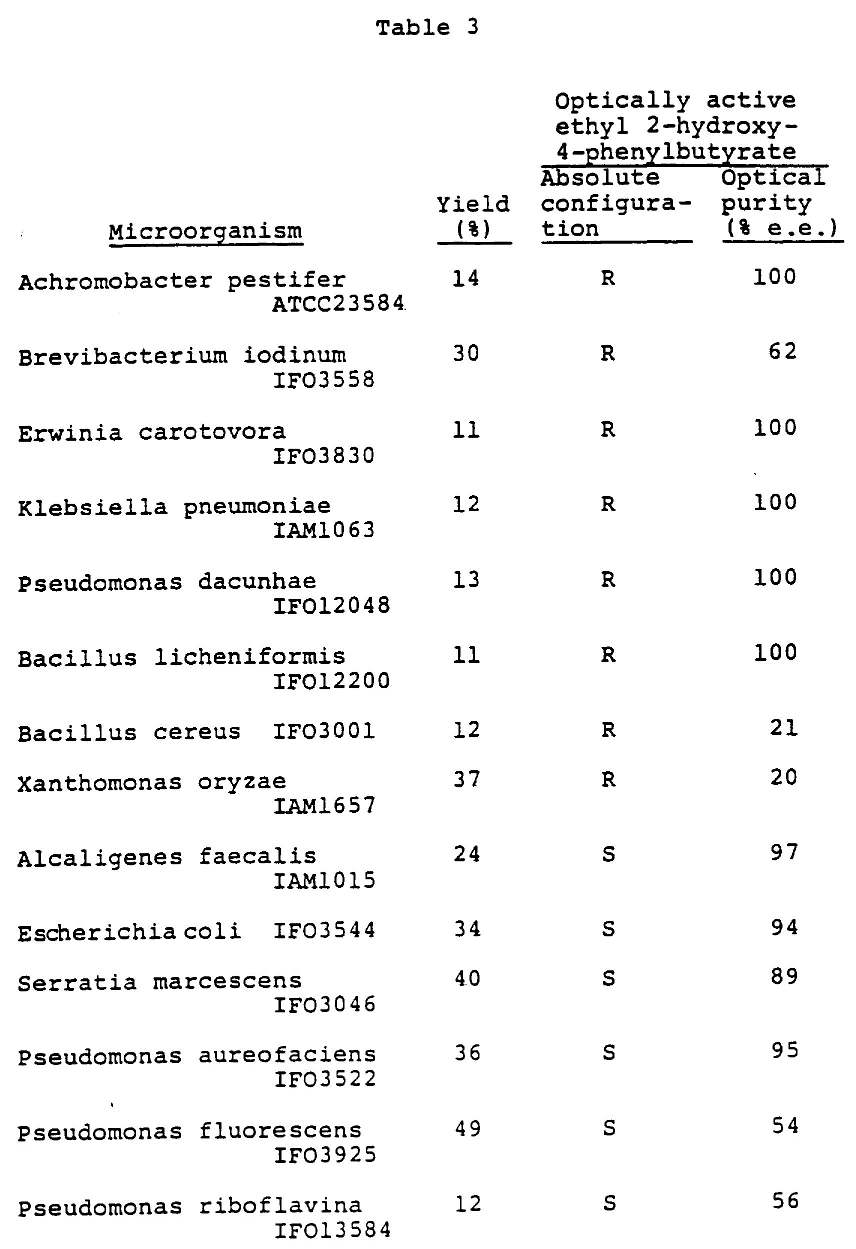

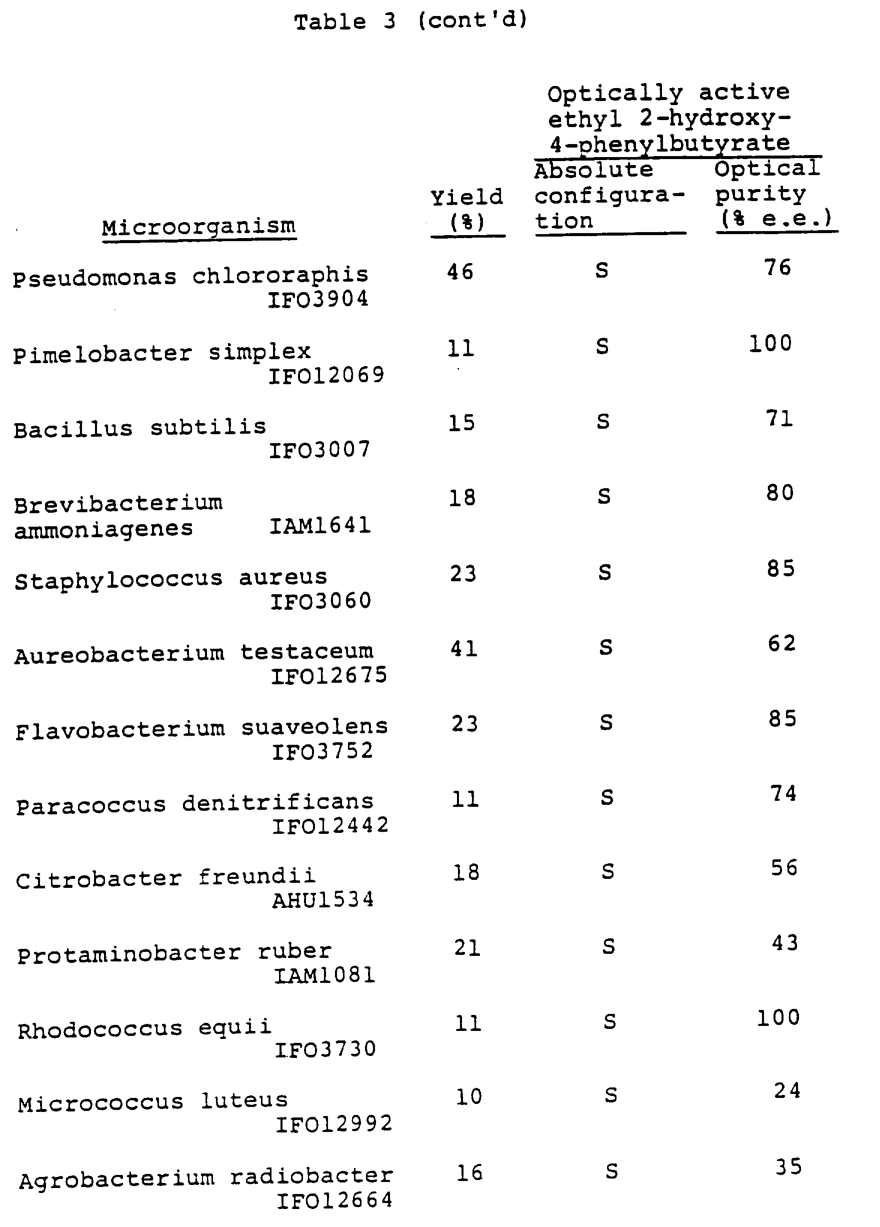

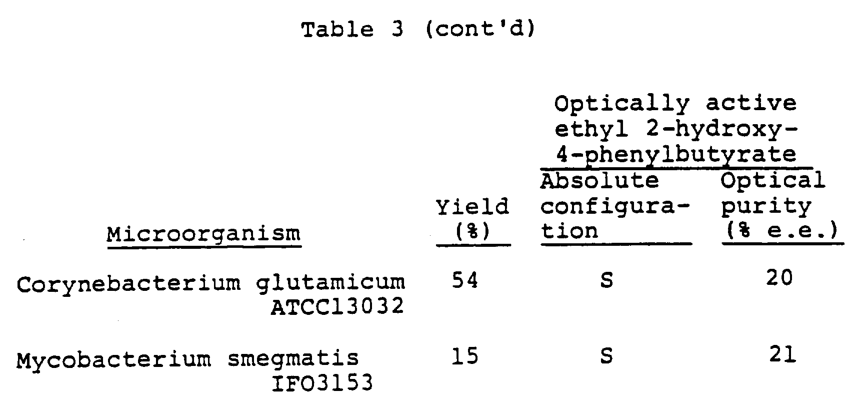

## Claims

1. A process for the production of an optically active 2-hydroxy acid derivative represented by the formula (II): 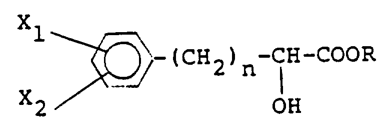
wherein X₁ and X₂ represent each a hydrogen atom, a halogen atom, a hyroxyl group, a nitro group or an alkyl group,
R represents an alkyl or a phenyl group, an
n is an integer of 0 to 3,
which comprises treating a 2-oxo acid derivative represented by the formula (I): 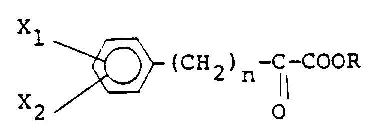
wherein X₁ and X₂ represent each a hydrogen atom, a halogen atom, a hydroxyl group, a nitro group or an alkyl group, R represents an alkyl or a phenyl group, and n is an integer of 0 to 3, with a microorganism which may be optionally ground, treated with acetone, lyophilized or immobilized, capable of asymmetrically reducing said 2-oxo acid derivative of the formula (I) into an optically active (R)- or (S)-form of said 2-hydroxy acid derivative of the formula (II) and recovering the optically active (R)- or (S)-2-hydroxy acid derivative of the formula (II) thus produced, said microorganism capable of producing said (R)-2-hydroxy acid derivative represented by the formula (II) being selected from among those belonging to the genera Streptococcus, Guilliermondella, Candida, Saccharomycopsis, Zygosaccharomyces, Sporidiobolus, Rhodosporidium, Saccharomyces, Schizosaccharomyces, Pichia, Issatchinkia, Rhodotorula, Kluyveromyces, Filobasidium, Torulaspora, Sporobolomyces, Hansenula, Lipomyces, Lodderomyces, Pachysolen, Saccharomycodes, Achromobacter, Brevibacterium, Erwinia, Klebsiella, Pseudomonas, Bacillus, Xanthomonas, Torulopsis and Chromobacterium and said microorganism capable of producing an (S)-2-hydroxy acid derivative of the formula (II) being selected from among those belonging to the genera Streptococcus, Sporolactobacillus, Ambrosiozyma, Botryoascus, Bretanomyces, Clavispora, Candida, Saccharomyces, Zygosaccharomyces, Schizosaccharomyces, Saccharomycopsis, Sporobolomyces, Rhodotorula, Pichia, Hansenula, Syringospora, Stephanoascus, Trigonopsis, Wickerhamiella, Wingea, Schwanniomyces, Geotrichum, Ashbya, Endomyces, Alcaligenes, Escherichia, Serratia, Pseudomonas, Pimelobacter, Bacillus, Brevibacterium, Staphylococcus, Aureobacterium, Flavobacterium, Paracoccus, Citrobacter, Protaminobacter, Rhodococcus, Micrococcus, Agrobacterium, Corynebacterium, Mycobacterium and Arthrobacter.

## Patentansprüche

1. Verfahren für die Herstellung eines optisch aktiven 2-Hydroxysäure-Derivats, dargestellt durch die Formel (II) : 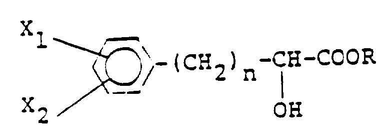
worin X₁ und X₂ jeweils ein Wasserstoffatom, ein Halogenatom, eine Hydroxylgruppe, eine Nitrogruppe oder eine Alkylgruppe bedeutet,
R eine Alkyl- oder eine Phenylgruppe bedeutet, und
n eine ganze Zahl von 0 bis 3 ist,
welches das Behandeln eines 2-Oxosäure-Derivats, dargestellt durch die Formel (I): 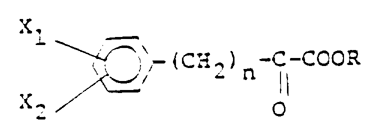
worin X₁ und X₂ jeweils ein Wasserstoffatom, ein Halogenatom, eine Hydroxylgruppe, eine Nitrogruppe oder eine Alkylgruppe bedeutet, R eine Alkyl- oder eine Phenylgruppe bedeutet und n eine ganze Zahl von 0 bis 3 ist, mit einem Mikroorganismus, welcher wahlweise gemahlen, mit Aceton behandelt, lyophilisiert oder immobilisiert sein kann und fähig ist, das besagte 2-Oxosäure-Derivat der Formel (I) in eine optisch aktive (R)- oder (S)-Form des besagten 2-Hydroxysäure-Derivats der Formel (II) asymmetrisch zu reduzieren, und das Gewinnen des so hergestellten optisch aktiven (R)- oder (S)-2-Hydroxysäure-Derivats der Formel (II) umfaßt, wobei der besagte Mikroorganismus, welcher fähig ist, das besagte, durch die Formel (II) dargestellte (R)-2-Hydroxysäure-Derivat zu produzieren, ausgewählt wird aus solchen, die zu den Gattungen Streptococcus, Guilliermondella, Candida, Saccharomycopsis, Zygosaccharomyces, Sporidiobolus, Rhodosporidium, Saccharomyces, Schizosaccharomyces, Pichia, Issatchinkia, Rhodotorula, Kluyveromyces, Filobasidium, Torulaspora, Sporobolomyces, Hansenula, Lipomyces, Lodderomyces, Pachysolen, Saccharomycodes, Achromobacter, Brevibacterium, Erwinia, Klebsiella, Pseudomonas, Bacillus, Xanthomonas, Torulopsis und Chromobacterium gehören und der besagte Mikroorganismus, der fähig ist, ein (S)-2-Hydroxysäure-Derivat der Formel (II) zu produzieren, ausgewählt wird unter solchen, die zu den Gattungen Streptococcus, Sporolactobacillus, Ambrosiozyma, Botryoascus, Bretanomyces, Clavispora, Candida, Saccharomyces, Zygosaccharomyces, Schizosaccharomyces, Saccharomycopsis, Sporobolomyces, Rhodotorula, Pichia, Hansenula, Syringospora, Stephanoascus, Trigonopsis, Wickerhamiella, Wingea, Schwanniomyces, Geotrichum, Ashbya, Endomyces, Alcaligenes, Escherichia, Serratia, Pseudomonas, Pimelobacter, Bacillus, Brevibacterium, Staphylococcus, Aureobacterium, Flavobacterium, Paracoccus, Citrobacter, Protaminobacter, Rhodococcus, Micrococcus, Agrobacterium, Corynebacterium, Mycobacterium und Arthrobacter gehören.

## Revendications

1. Procédé de production d'un dérivé d'acide 2-hydroxy optiquement actif représenté par la formule (II): 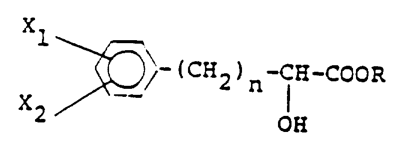
dans laquelle X₁ et X₂ représentent chacun un atome d'hydrogène, un atome d'halogène, un groupe hydroxyle, un groupe nitro ou un groupe alkyle,
R représente un groupe alkyle ou un groupe phényle, n est un nombre entier de 0 à 3,
qui comprend le traitement d'un dérivé de 2-oxacide représenté par la formule (I): 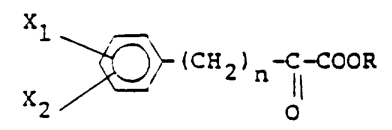 dans laquelle X₁ et X₂ représentent chacun un atome d'hydrogène, un atome d'halogène, un groupe hydroxyle, un groupe nitro ou un groupe alkyle,
R représente un groupe alkyle ou un groupe phényle, n est un nombre entier de 0 à 3,
avec un micro-organisme qui peut éventuellement être moulu, traité avec de l'acétone, lyophilisé ou immobilisé, capable de réduire de manière asymétrique ledit dérivé de 2-oxacide représenté par la formule (I) en une forme (R) ou (S) optiquement active dudit dérivé d'acide 2-hydroxy représenté par la formule (II) et la récupération du dérivé de l'acide (R)- ou (S)-2-hydroxy optiquement actif représenté par la formule (II) ainsi produit, ledit micro-organisme capable de produire ledit dérivé d'acide (R)-2-hydroxy de formule (II) étant choisi parmi ceux appartenant aux genres Streptococcus, Guilliermondella, Candida, Saccharomycopsis, Zygosaccharomyces, Sporidiobolus, Rhodosporidium, Saccharomyces, Schizosaccharomyces, Pichia, Issatchinkia, Rhodotorula, Kluyveromyces, Filobasidium, Torulaspora, Sporobolomyces, Hansenula, Lipomyces, Lodderomyces, Pachysolen, Saccharomycodes, Achromobacter, Brevibacterium, Erwinia, Klebsiella, Pseudomonas, Bacillus, Xanthomonas, Torulopsis et Chromobacterium et ledit micro-organisme capable de produire ledit dérivé d'acide (S)-2-hydroxy de formule (II) étant choisi parmi ceux appartenant aux genres Streptococcus, Sporolactobacillus, Ambrosiozyma, Botryoascus, Bretanomyces, Clavispora, Candida, Saccharomyces, Zygosaccharomyces, Schizosaccharomyces, Saccharomycopsis, Sporobolomyces, Rhodotorula, Pichia, Hansenula, Syringospora, Stephanoascus, Trigonopsis, Wickerhamiella, Wingea, Schwanniomyces, Geotrichum, Ashbya, Endomyces, Alcaligenes, Escherichia, Serratia, Pseudomonas, Pimelobacter, Bacillus, Brevibacterium, Staphylococcus, Aureobacterium, Flavobacterium, Paracoccus, Agrobacterium, Flavobacterium, Paracoccus, Citrobacter, Protaminobacter, Rhodococcus, Micrococcus, Agrobacterium, Corynebacterium, Mycobacterium ou Arthrobacter.
